# FASCICULE DE BREVET EUROPEEN

(11) **EP 1 075 256 B1**
(45) Date de publication et mention de la délivrance du brevet: **30.07.2003**
(21) Numéro de dépôt: 99916992.3
(22) Date de dépôt: 05.05.1999
(51) Int. Cl.: A61K 31/05, A61K 31/70, A61P 43/00

(54) **ANTAGONISTES DES LIGANDS DU RECEPTEUR DES ARYLHYDROCARBURES**
ANTAGONISTE DER ARYLKOHLENWASSERSTOFF-REZEPTOREN LIGANDEN
ARYLHYDROCARBON RECEPTOR LIGAND ANTAGONISTS

(30) Priorité: 05.05.1998 FR 9805673
(43) Date de publication de la demande: 14.02.2001
(73) Titulaire: INSTITUT NATIONAL DE LA SANTE ET DE LA RECHERCHE MEDICALE (INSERM), 75654 Paris Cédex 13 (FR)
(72) Inventeur: SAVOURET, Jean-François, F-94240 L'Hay-les-Roses (FR); CASPER, Robert-Frédéric, F-75007 Paris (FR); MILGROM, Edwin, F-92330 Sceaux (FR)
(74) Mandataire: Peaucelle, Chantal
(86) Numéro de dépôt international: FR9901063
(87) Numéro de publication internationale: WO99056737

(56) Documents cités:
- EP-A- 0 773 020
- WO-A-99/04747
- US-A- 3 624 162
- CIOLINO, HENRY P. ET AL: "Resveratrol inhibits transcription of CYP1A1 in vitro by preventing activation of the aryl hydrocarbon receptor" CANCER RES. (1998), 58(24), 5707-5712 CODEN: CNREA8;ISSN: 0008-5472, XP002090260
- JANG, MEISHIANG ET AL: "Cancer chemopreventive activity of resveratrol, a natural product derived from grapes" SCIENCE (WASHINGTON, D. C.) (1997), 275(5297), 218-220 CODEN: SCIEAS;ISSN: 0036-8075, XP002083956
- DATABASE WPI Section Ch, Week 9409 Derwent Publications Ltd., London, GB; Class B05, AN 94-071830 XP002090269 & JP 06 024967 A (YUSHIRO CO), 1 février 1994 (1994-02-01)
- DATABASE WPI Section Ch, Week 9517 Derwent Publications Ltd., London, GB; Class B05, AN 95-128236 XP002090270 & JP 07 053359 A (KAO CORP), 28 février 1995 (1995-02-28)
- PATENT ABSTRACTS OF JAPAN vol. 096, no. 011, 29 novembre 1996 (1996-11-29) & JP 08 175960 A (KAO CORP), 9 juillet 1996 (1996-07-09)
- DATABASE WPI Section Ch, Week 9810 Derwent Publications Ltd., London, GB; Class B04, AN 98-105073 XP002090271 & JP 09 328410 A (MITSUBA BOEKI KK), 22 décembre 1997 (1997-12-22)
- DATABASE WPI Section Ch, Week 9749 Derwent Publications Ltd., London, GB; Class B04, AN 97-527396 XP002090272 & CN 1 127 070 A (HOU R), 24 juillet 1996 (1996-07-24)
- DATABASE WPI Section Ch, Week 8637 Derwent Publications Ltd., London, GB; Class B04, AN 86-242358 XP002090273 & JP 61 171427 A (OSAKA YAKUHIN KENKYUSHO KK), 2 août 1986 (1986-08-02)
- G.Y. SUN ET AL.: "Protective role of polyphenols against neurodegeneration" JOURNAL OF NEUROCHEMISTRY, vol. 70, no. s1, mars 1998 (1998-03), page s59 XP002090261
- DRACZYNSKA-LUSIAK, BOZENA ET AL: "Oxidized lipoproteins may play a role in neuronal cell death in Alzheimer disease" MOL. CHEM. NEUROPATHOL. (1998), 33(2), 139-148 CODEN: MCHNEM;ISSN: 1044-7393, XP002090262
- DRACZYNSKA-LUSIAK, B. ET AL: "Oxidized lipoproteins activate NF-.kappa.B binding activity and apoptosis in PC12 cells" NEUROREPORT (1998), 9(3), 527-532 CODEN: NERPEZ;ISSN: 0959-4965, XP002090263
- KIMURA, YOSHIYUKI ET AL: "Effects of stilbenes on arachidonate metabolism in leukocytes" BIOCHIM. BIOPHYS. ACTA (1985), 834(2), 275-8 CODEN: BBACAQ;ISSN: 0006-3002, XP002090264
- TSURUGA, TOMOKO ET AL: "Biologically active constituents of Melaleuca leucadendron: inhibitors of induced histamine release from rat mast cells" CHEM. PHARM. BULL. (1991), 39(12), 3276-8 CODEN: CPBTAL;ISSN: 0009-2363, XP002090265
- I.O. GODFROID: "L'éloge du vin ?" PRESSE MEDICALE, vol. 26, no. 40, 1997, pages 1971-1974, XP002090266
- GEHM, BARRY D. ET AL: "Resveratrol, a polyphenolic compound found in grapes and wine, is an agonist for the estrogen receptor" PROC. NATL. ACAD. SCI. U. S. A. (1997), 94(25), 14138-14143 CODEN: PNASA6;ISSN: 0027-8424, XP002090267
- MAZUMDER, ABHIJIT ET AL: "Effects of Tyrphostins, Protein Kinase Inhibitors, on Human Immunodeficiency Virus Type 1 Integrase" BIOCHEMISTRY (1995), 34(46), 15111-22 CODEN: BICHAW;ISSN: 0006-2960, XP002090268

## Description

L'invention a pour objet des antagonistes du récepteur des arylhydrocarbures polycycliques et/ou halogénés toxiques et leurs applications, notamment, pour la prévention et le traitement des intoxications et pathologies induites par ces polluants.

Les arylhydrocarbures polycycliques et/ou halogénés, désignés ci-après en abrégé par ligands AhR, constituent des toxiques environnementaux. Il s'agit par exemple d'hydrocarbures halogénés (HAHs), d'hydrocarbures polyaromatiques halogénés (PAHs), de biphényles polychlorés (PCBs), de polychlorodibenzodioxines (PCDD), dont la 2, 3, 7, 8-tétrachlorodibenzo (p)dioxine (TCDD), de polychloro-dibenzofuranes (PCDF), et de benzo(a)pyrène (BaP).

Ces ligands AhR, et en particulier la dioxine, ont récemment reçu beaucoup d'attention du fait de leur résistance à la dégradation et de leur longue demi-vie de plus de 10 ans dans le sol et de 4 à 12 ans dans le corps humain (sang et graisses).

Contrairement à une croyance répandue, l'exposition aux ligands AhR ne se limite pas à la vicinité des sites industriels, de décharge, ou d'accidents de pollution. Toute personne sur terre est continuellement exposée aux toxines activatrices du AhR provenant, entre autres, de la fumée de cigarette, des gaz d'échappement des moteurs à essence ou diesel, des émanations des fours industriels, de la viande cuite au barbecue, des produits laitiers et même du lait maternel (voir référence (1) ; les références bibliographiques sont données en fin de description).

Il existe maintenant suffisamment de preuves expérimentales et cliniques pour démontrer un lien entre l'exposition aux ligands AhR et de nombreuses pathologies, telles que l'athérosclérose (2), certains cancers (3), (4), (5), la dépression immunitaire (6), les allergies et certaines maladies de la peau (7).

En outre, les ligands AhR pourraient être impliqués dans l'étiologie de certaines stérilités ( (8), (9) ), et de la diminution de la résistance aux infections virales ( (10) (11) ).

Les effets biologiques des ligands AhR sont médiés par le récepteur des arylhydrocarbures (AhR), une protéine de 108 kDa, membre de la famille bHLH/PAS, qui est présente dans le cytosol des cellules de mammifères, pratiquement dans tous les tissus (pour revue: (12) ).

Le complexe ligand/AhR est ensuite transporté dans le noyau par association avec une protéine nucléaire, ARNT ou "AhR nuclear translocator". Dans le noyau, ce complexe hétéromérique régule l'expression de certains gènes en se liant à l'ADN au niveau de séquences définies, les éléments de réponse à la dioxine (DRE), ou les éléments de réponse xénobiotiques (XRE), qui répondent à la séquence T/GNGCGTG. Ces éléments sont localisés dans les gènes sensibles comme les cytochromes P-450 CYP1A1, CYP1A2, CYP1B1, dénommés gènes de phase I.

Ces protéines, en particulier CYP1A1 et CYP1B1, sont impliquées dans le métabolisme des HAHs, PAHs et des dioxines, souvent pour conduire à des composés plus cancérigènes.

De plus l'induction de CYP1A1/AHH entraîne une augmentation de la production de radicaux libres oxygénés (ROS) qui provoquent la peroxydation des lipides, des lésions oxydatives de l'ADN et des tissus, et qui peuvent conduire à des phénomènes pathologiques de tératogénèse et carcinogénèse (13).

Les ligands AhR induisent aussi les enzymes dits de phase II (glutathion-S-transférase, aldéhyde-3-déhydrogénase, NAD(P)H quinone réductase), qui sont responsables de la détoxification et de l'excrétion des molécules xénobiotiques. Cette induction semble avoir lieu par un mécanisme indirect mettant en jeu des éléments de réponse aux anti-oxydants dans les gènes concernés.

Ainsi les ligands AhR induisent une réponse transcriptionnelle complexe qui peut simultanément provoquer des effets pathologiques et concourir à l'élimination des toxiques.

Les inventeurs ont entrepris d'identifier de nouveaux ligands du récepteur AhR. Pour ce faire, ils ont transfecté de façon stable une lignée de cellules de cancer du sein, T-47D, avec un gène rapporteur (la trans-acétylase du chloramphénicol, ou CAT) placé sous le contrôle d'un élément de réponse à la dioxine ou DRE. La lignée T-47D est connue pour contenir un taux élevé de récepteur AhR (170 femtomoles/mg protéines).

Une cinquantaine de molécules ont été sélectionnées sur la base de similitude structurale globale ou partielle avec la dioxine. Ces travaux ont montré la capacité de certains composés polyhydroxylés aromatiques à bloquer de manière spécifique la liaison des ligands AhR au récepteur AhR et l'induction consécutive des enzymes de phase I par lesquels les AhR exercent leurs effets nocifs.

La plupart des composés ne se sont révélés ni agonistes, ni antagonistes de la dioxine. Ceux présentant une activité agoniste, exepté la quercétine, étaient en revanche dépourvus d'activité antagoniste.

On mesurera donc l'intérêt des propriétés spécifiquement antagonistes des composés polyhydroxylés évoqués ci-dessus.

Mettant à profit le mécanisme de compétition pour la liaison au récepteur AhR entre ces composés et les ligands AhR, l'invention a pour but de fournir des antagonistes de ce récepteur.

Elle vise également les applications de ces composés polyhydroxylés, chez l'homme et l'animal, notamment dans les domaines thérapeutiques, alimentaires et, de manière générale, pour prévenir ou traiter les affections dues aux effets toxiques résultant d'une exposition aux ligands AhR.

L'invention vise ainsi l'utilisation de stilbènes polyhydroxylés, monomères ou polymères, ou de leurs glycosides, ces composés étant sous forme racémique, ou d'isomères géométriques, pour la fabrication d'antagonistes des ligands du récepteur des arylhydrocarbures, pour traiter les pathologies induites par ces polluants.

L'étude de ces composés par les inventeurs a en effet montré qu'ils sont capables d'inhiber les effets transcriptionnels des ligands AhR par un mécanisme de compétition pour la liaison au récepteur AhR.

On dispose ainsi de produits à effet anti-dioxine qui constituent donc des agents anti-pollution.

Dans un mode préféré de réalisation de l'invention, lesdits stilbènes sont trihydroxylés, et en particulier sont constitués par les 3, 5, 4' - trihydroxystilbènes ou resvératrols, et notamment par le trans-resvératrol.

Dans un autre mode de réalisation de l'invention, lesdits stilbènes sont tetrahydroxylés. Des composés avantageux comprennent le 3, 4, 3', 5-tétrahydroxystilbène ou picéatannol et le 2, 3', 4, 5'- tétrahydroxystilbène ou oxyresvératrol.

Dans encore un autre mode de réalisation de l'invention, lesdits stilbènes sont dihydroxylés et comprennent, avantageusement, les 4, 4' - dihydro-xystilbènes.

Les glycosides des stilbènes polyhydroxylés sont avantageusement des glucosides, des galactosides, des lactosides, des mannosides, des fructosides ou des picéosides. Le résidu sucre est sous forme monomère ou polymère.

Des travaux antérieurs avaient porté sur l'étude du rôle de polyphénols présents dans le vin, dont le resvératrol, dans divers processus pathologiques.

Des activités anti-cyclooxygénase, antioxydantes et anticancéreuse ont été évoquées, mais n'ont pas permis de déboucher sur l'élaboration de formes galéniques permettant la mise au point de traitements efficaces.

On mesurera alors l'originalité de l'invention basée sur la mise en évidence d'un mécanisme complètement nouveau pour ces dérivés, l'inhibition du récepteur AhR, et qui permet le développement d'applications dans de nombreux domaines, et tout spécialement dans un contexte polluant.

Ainsi, l'étude des antagonistes de l'invention a montré qu'ils sont capables d'inhiber *ex vivo* et *in vivo* l'induction de gènes par les ligands AhR et les pathologies qui lui sont liées.

En particulier, ces antagonistes sont capables de bloquer l'induction des enzymes de phase I par les ligands et de manière générale de protéger contre des effets transcriptionnels pathologiques liés à l'exposition à ces substances nocives.

L'invention vise donc leur utilisation pour la prévention et le traitement d'affections provoquées par l'exposition à des ligands AhR.

A ce titre, l'invention vise l'utilisation des antagonistes définis ci-dessus pour la fabrication de médicaments destinés à la prévention et au traitement de pathologies induites ou favorisées (dans le sens d'une aggravation d'une maladie préexistante), plus spécialement liées à une exposition aux arylhydrocarbures polycycliques et/ou halogénés.

Ces médicaments renferment le ou lesdits antagonistes en une quantité thérapeutiquement efficace et permettant d'obtenir l'effet antagoniste recherché vis-à-vis du récepteur d'arylhydrocarbures, en association avec un véhicule ou excipient pharmaceutiquement acceptable.

Dans l'utilisation selon l'invention, les médicaments se présentent sous une forme pour l'administration par voie orale, nasale, parentérale topique.

Les médicaments ainsi élaborés se présentent sous des formes appropriées pour l'administration par voie orale, telles que gouttes, gélules, sirop, sirop alcoolique, par voie nasale, telle que spray ou gouttes, par voie parentérale, sous forme de solution dans un solvant approprié, ou encore par voie topique, telle que crème, pommade, shampooing ou lotion. Les véhicules ou excipients pharmaceutiquement acceptables utilisés sont à base d'huiles admises à la pharmacopée, ou d'un alcool comme l'éthanol (de 10 % à 100 %) ou de DMSO. De manière générale, il s'agit de solvants organiques non aqueux. On peut également avoir recours à une préparation lipidique telle que Intralipid ^{R}.

On notera que lesdits stilbènes antagonistes sont solubles dans les excipients et/ou véhicules utilisés pour l'élaboration des médicaments.

La posologie dans les différentes formes et pour l'application quotidienne, sera établie, de manière classique, selon le type d'effets à traiter.

A titre d'exemple, on administrera lesdits médicaments à base desdits antagonistes à raison de 0,1 mg à 5 g/jour, notamment de 20 mg à 200 mg/jour, en particulier de 10 à 100 mg/jour.

D'une manière avantageuse, les stilbènes polyhydroxylés utilisés, selon l'invention, comme antagonistes, présentent une grande innocuité et ne donnent pas lieu à des effets secondaires nocifs.

Les médicaments élaborés à partir de ces antagonistes sont utilisables dans diverses pathologies impliquant des ligands AhR, comme l'athérogénèse, l'immunosuppression, le cancer, les infections virales telles que le SIDA, les allergies et maladies dermatologiques associées à la dioxine et aux ligands AhR ainsi qu'à l'ostéoporose.

Divers travaux ont montré le rôle joué par les ligands AhR dans ces pathologies.

Ainsi, il a été démontré que, dans le système cardiovasculaire, les ligands AhR peuvent présenter des propriétés athérogènes par la perturbation des fonctions des cellules endothéliales des vaisseaux sanguins. Les ligands AhR induisent CYP1A1/AHH dans les cellules endothéliales de porc avec une DE₅₀ (dose induisant 50% du phénomène) de 180 nM pour le BaP et 0.015 nM pour la TCDD (17). La stimulation de ces enzymes de phase I provoque une augmentation de la production de ROS (13) capable de provoquer des lésions membranaires dans les cellules endothéliales et la peroxydation des lipides. Les LDL oxydées formées par ce mécanisme font l'objet d'une endocytose non régulée par un récepteur membranaire dédié qui conduit à une hyperaccumulation dans les cellules endothéliales et à la formation de cellules d'aspect "mousse" (18).

Outre les ligands AhR, l'acide arachidonique et les eicosanoides vasoactifs sont aussi des substrats pour CYP1A1/AHH. Il a été proposé que c'est entre autres par la perturbation du métabolisme de l'acide arachidonique que les ligands AhR pourraient être athérogènes (19). Dans (2), les auteurs ont aussi montré que les ligands AhR peuvent léser les cellules endothéliales et modifier leur perméabilité, ce qui pourrait conduire à une incorporation accrue de déchets des cholestérol-lipoprotéines dans la paroi artérielle conduisant à la formation d'athérome. Dans le cas des PCBs, cet effet n'est observé qu'avec les congénères qui lient AhR et induisent CYP1A1. De plus, ces mêmes PCBs augmentent le stress oxydatif et la peroxydation des lipides dans les cellules endothéliales en culture, phénomène qui a été corrélé dans le temps avec l'induction de CYP1A1 et qui pourrait être le mécanisme des lésions cellulaires.

On a également rapporté que les ligands AhR, comme la dioxine, sont de puissants immunosuppresseurs (6) et sont tumorigènes chez les rongeurs. A la suite de l'immunosuppression par les ligands AhR, on observe une diminution de la résistance aux agents infectieux (11). Les ligands AhR peuvent induire l'immunosuppression par de nombreux mécanismes: (20) à (28).

Le rôle des ligands AhR dans l'immunosuppression est soutenu par la présence du récepteur AhR, de ARNT ainsi que du gène inductible CYP1A1/AHH dans les lymphocytes (23).

Les ligands AhR sont également connus pour stimuler la réplication des virus (29). Il est généralement admis que l'activation des virus VIH-1 latents par les ligands AhR est un phénomène important dans la progression du syndrome humain d'immunodéficience acquise (SIDA) (29) et (10). Les ligands AhR activent l'expression du VIH-1 par l'induction de CYP 1A1 ainsi que par l'activation du facteur NF-kappa B (29) et (10). Les gènes de ces deux protéines contiennent des sites DRE médiant l'activation du gène par le récepteur AhR, de même que le promoteur présent dans le LTR (long terminal repeat) du virus VIH. De plus, les deux voies de pénétration du VIH dans les lymphocytes, les protéines membranaires CXCR4 et CD4 contiennent aussi des DRE dans les promoteurs de leurs gènes (voir tableau 2 dans les exemples).

Les ligands AhR sont également responsables d'allergies et de maladies dermatologiques. Les hydrocarbures et la dioxine présents dans la fumée d'échappement de moteur diesel augmentent la production d'IgE par les lymphocytes B (30), ce qui contribue au développement d'affections allergiques de la peau et des voies respiratoires. De plus, le gène de l'immunoglobuline E contient un DRE (tableau 2). La surproduction d'IgE est un trait caractéristique des phénomènes d'allergie.

Les ligands AhR sont de puissants inducteurs de la chloracné. Dans tous les accidents industriels où ces composés ont été impliqués, (RFA, 1953; USA, 1964; Japon, 1968; Italie, 1976; Chine, 1977-79), la chloracné fut considérée comme le symptôme spécifique majeur de l'intoxication (7) et fut observée chez la majorité des personnes exposées. Cette éruption acnéiforme s'observe à la suite d'une altération des processus de différenciation des cellules basales des glandes sébacées et des kératinocytes (31). Ces cellules expriment des taux croissants du récepteur AhR durant leur différenciation (32). De plus, les ligands AhR eux-même augmentent la différenciation terminale des kératinocytes (31). Les ligands Ahr doivent alors induire des modifications cutanées spécifiques en activant le récepteur AhR, ce qui conduit à la modification de l'expression des gènes sensibles dans la peau. Enfin, de nombreuses données épidémiologiques montrent que le tabagisme est un facteur de risque de l'ostéoporose chez l'homme comme chez la femme et (33). Ceci est confirmé par la relation proportionnelle quantitative qui existe entre le tabagisme et la diminution de la densité osseuse (34), 35 et (36) et par la corrélation entre tabagisme et risques de fractures (34), (37). Les ligands AhR présents dans le tabac pourraient exercer un effet négatif sur l'os de trois manières: 1) effet anti-estrogénique direct (voir (38) pour revue); 2)effet toxique direct sur l'os puisque le tissu osseux contient des récepteurs AhR (39) ; 3) effet toxique direct sur la fonction ovarienne: Les femmes qui fument font une ménopause plus précoce de 1 à 4 ans que que celle ne fumant pas. Des expériences sur animaux démontrent un effect toxique direct du benzo(a)pyrène et des autres ligands AhR sur les oocytes.

De plus, la présence constante de sites DRE dans les gènes impliqués dans l'inflammation tels que IgE, cytokines, chemokines, leurs récepteurs ainsi que la NO synthase inductible, suggère un rôle du récepteur AhR et de ses ligands dans les pathologies inflammatoires provoquées par une production excessive d'IgE et/ou d'oxyde nitreux NO par les cellules mononucléaires infiltrées dans les tissus à la suite de la sécrétion de chemokines chimio-attractives par ces tissus. On peut citer par exemple les dermatites atopiques, l'arthrite rhumatoïde, les maladies neurodégénératives (sclérose en plaque, sclérose amyotrophique), diabète, et avortements à répétition.

La présence de DRE dans les gènes des chemokines et/ou de leurs récepteurs renforce le lien entre ligands AhR, inflammation et états fébriles. La fièvre est provoquée par l'interaction de pyrogènes bactériens ou viraux avec les cellules immunocompétentes conduisant à la sécrétion d'une cascade de cytokines. Les cytokines dont l'association avec les états fébriles a été démontrée sont le TNF-a, IL-1, IL-6, IL-8 et le Macrophage Inflammatory Peptide ( MIP-1). En outre, la fièvre périodique de la malaria semble être médiée par la sécrétion de TNF et de MIP-1 sous le contrôle de l'hémozoine. Le tableau 4 montre que les gènes codant pour les récepteurs de IL-1, IL-8 et RANTES/MIP-1 contiennent tous des DRE dans leur régions promotrices. Les préparations pharmaceutiques de l'invention pourraient alors jouer un rôle modérateur au cours des fièvres induites par l'interaction de ces cytokines avec leurs récepteurs.

En dernier lieu, la présence de sites DRE dans la séquence codante de la protéine PrP (ou prion de la maladie de Creutzfeld-Jacob) chez l'humain comme chez les bovins et dans le promoteur du gène de la préséniline (maladie d'Alzheimer) suggère que les ligands AhR puissent induire ou aggraver ces maladies neurodégénératives. En contrepartie, ces maladies pourraient être améliorées par administration de médicaments selon l'invention.

L'invention vise donc l'utilisation desdits antagonistes pour la fabrication de médicaments anti-viraux pour prévenir ou réduire les troubles associés aux infections virales induites ou favorisées par les ligands AhR, comme le rhume ou la grippe, ou pour traiter les polyposes nasales, pour traiter l'activation des infections virales latentes par les ligands AhR, telle que le SIDA induit par VIH.

L'invention vise également leur utilisation pour la prévention de l'encéphalite spongiforme causée par la protéine PrP (prion) chez l'humain et les bovins.

L'invention vise encore leur utilisation pour la prévention de l'ostéoporose chez la femme à tous les âges de la vie et en particulier en post-ménopause et chez la femme âgée.

Elle vise en outre leur utilisation pour le traitement des états inflammatoires causés par la production excessive d'oxyde nitreux et/ou d'IgE, notamment de dermatites atopiques, arthrite rhumatoïde, ostéo-arthrite, les maladies neurodégénératives (sclérose en plaques, sclérose amyotrophique) et diabète.

L'invention est également appropriée pour la fabrication de médicaments pour le traitement de dermatites, acné, lésions d'hyperkératose, eczéma, ainsi que pour le vieillissement de la peau et les rides.

L'utilisation desdits antagonistes pour la fabrication de médicaments anti-viraux pour réduire les états fébriles associés aux maladies infectieuses, virales et parasitaires entre également dans le champ de l'invention.

De même l'invention concerne l'utilisation de ces antagonistes pour la fabrication de médicaments pour le traitement des pathologies gynécologiques et endocriniennes telles que l'endométriose, les fibromes (léiomyomes), la pré-éclampsie et les avortements à répétition.

L'invention a également pour objet l'utilisation desdits antagonistes pour la fabrication de médicaments pour la prévention et le traitement de la maladie d'Alzheimer.

Les effets inhibiteurs desdits stilbènes vis-à-vis des ligands AhR sont également avantageusement mis à profit en diététique.

L'invention vise également l'utilisation des antagonistes définis ci-dessus comme additifs alimentaires.

Elle vise ainsi des aliments caractérisés en ce qu'ils renferment au moins un antagoniste tel que défini ci-dessus en une quantité lui permettant d'exercer un effet inhibiteur vis-à-vis de ligands AhR, de manière à prévenir leurs effets nocifs. Ces aliments sont destinés à l'adulte ou à l'enfant.

Ces additifs sont incorporés dans l'alimentation, par exemple dans le lait en poudre, les préparations liquides ou solides (céréales) ou les aliments en conserve.

L'incorporation peut être réalisée durant l'élaboration des aliments ou lors de leur conditionnement, ou en utilisant des préparations avec les antagonistes déjà formulées, permettant d'effectuer la supplémentation désirée.

Les antagonistes utilisés selon l'invention sont particulièrement appropriés pour une supplémentation diététique de l'alimentation des enfants en bas âge nouveaux nés, nourrissons et très jeunes enfants dans un contexte d'exposition aux polluants aryhydrocarbures polycycliques et /ou halogénés.

La dose utilisée sera réduite par rapport à celle retenue pour un adulte, en tenant compte du poids de l'enfant et de son alimentation, de manière à obtenir une concentration plasmatique efficace (de l'ordre du micromolaire).

Les antagonistes de l'invention présentent également un grand intérêt pour lutter contre les effets du tabagisme, qui correspond à une exposition chronique aux ligands AhR.
Les ligands AhR sont présents dans la fumée de cigarette, en particulier des polychloro-dibenzo-p-dioxines (PCDD), dont la dioxine (TCDD), et des PAHs, dont le benzo(a)pyrène (BaP). La somme de tous ces produits est équivalente à 4,3 pg/kg de poids corporel pour 20 cigarettes par jour. Chez le grand fumeur, la charge peut aller jusqu'à 0,05 mg/kg. Tous ces composés présents dans la fumée de cigarette sont capables d'induire l'expression de CYP1A1/AHH dans les poumons (4), le placenta, les reins (41), les ovaires et les cellules endothéliales des vaisseaux sanguins. L'induction de CYP1A1/AHH et des autres enzymes de la phase I conduit à la production de ROS qui provoquent des lésions oxydatives sur l'ADN (43). La métabolisation des hydrocarbures polyaromatiques par CYP1A1/AHH résulte en outre en la production d'intermédiaires carcinogènes très réactifs (43) qui se lient à l'ADN pour former des adducts covalents.

Le tabagisme a été mis en cause directement dans l'occurrence d'au moins huit types différents de cancers humains dans le poumon, la vessie, le tractus intestinal, et les lymphocytes (pour revue, voir (44)). Les ligands AhR ont été impliqués dans la génèse du cancer du poumon, par l'identification d'un polymorphisme génétique de CYP1A1. En outre, le tabagisme est une cause directe de l' ischémie cardiaque, la principale cause de mortalité dans le monde occidental (44). Il existe aussi des données épidémiologiques qui relient le tabagisme à l'accélération du SIDA (10) ainsi qu'à l'ostéoporose (33), (34), (35).

On met à profit les propriétés avantageuses des antagonistes de l'invention en les utilisant pour imprégner les filtres de cigarette, afin de délivrer une dose d'antagoniste proportionnelle à la concentration de toxiques absorbée avec la fumée, et conduisant à une concentration sanguine permettant de bloquer l'induction de CYDP1A1 et des autres enzymes de phase I au niveau des organes en contact avec les arylhydrocarbures (voies respiratoires, poumon, rein, vessie).

Les stilbènes polyhydroxylés utilisés selon l'invention sont obtenus par extraction à partir de végétaux, notamment en ce qui concerne les glycosides, ou par voie de synthèse. Les isomères géométriques, lorsqu'ils existent sont séparés selon les techniques classiques.

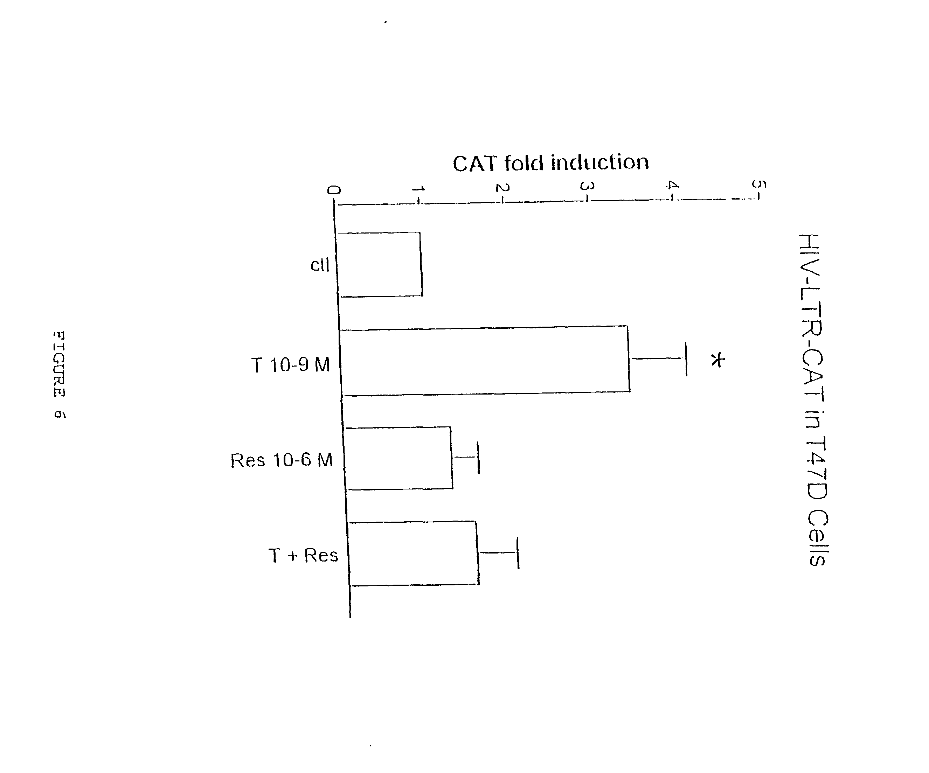

D'autres caractéristiques et avantages de l'invention sont donnés dans les exemples qui suivent qui se rapportent, à titre illustratif, au trans-resvératrol, qui sera appelé Resvératrol. Dans ces exemples, les figures 1 à 7 représentent :
- les figures 1A et 1B, les courbes d'induction (nombre de fois) d'un gène rapporteur placé sous le contrôle d'un élément de réponse à la dioxine, transfecté dans la lignée T-47 D, en fonction de la concentration en TCDD, en présence de TCDD seule ou de TCDD+ Resvératrol (1A), et d'inhibition de l''induction par TCDD (en %) de ce gène en fonction de la concentration en Resvératrol, (1B),
- la figure 2, l'inhibition par le Resvératrol de l'induction de CYp1A1 par TCDD dans les cellules T-47D,
- la figure 3, les courbes de compétition de liaison au récepteur AhR entre TCDD, αNF, le Resvératrol, l'indole-3-carbinol, dans le cytosol de foie de lapin,
- la figure 4, l'activité estrogénique de l'oestradiol comparé à celle du Resvératrol, à différentes concentrations, sur un gène rapporteur oestrogéno-dépendant transfecté de façon transitoire dans les cellules T-47D,
- la figure 5, la régulation de l'activité NAD(P)H quinone réductase par TCDD et ses antagonistes dans les cellules T-47D,
- la figure 6, la répression de l'induction du promoteur du LTR de VIH (VIH-LTR-CAT) par TCDD dans les cellules T-47D, et
- la figure 7, l'inhibition de l'induction *in vivo* de Cyp 1A1 chez le rat par l'association benzo [a] pyrène/DMBA.

### . Inhibition de l'induction de la transcription des enzymes de phase I par les ligands AhR.

On transfecte de manière stable la lignée T-47D de cellules de cancer du sein avec un gène rapporteur, la trans-acétylase du chloramphénicol, CAT en abrégé, placé sous le contrôle d'un élément de réponse à la dioxine.

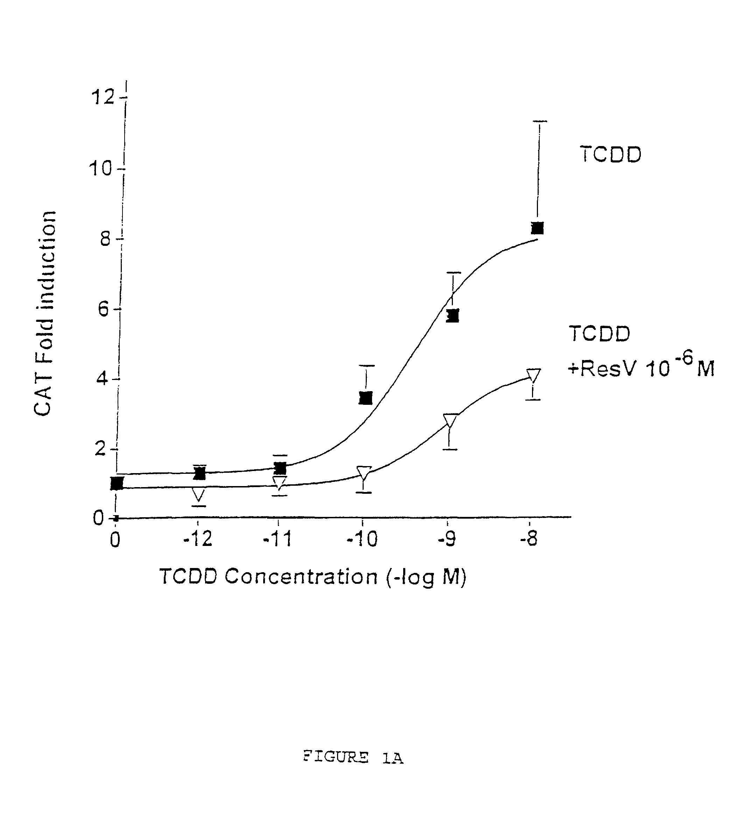

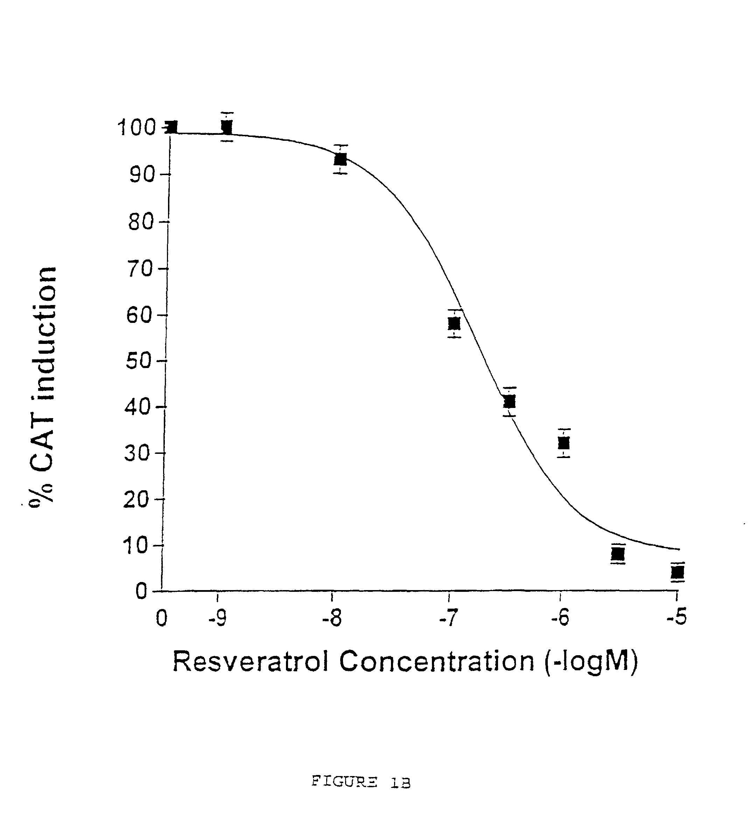

On rapporte sur la figure 1A, en fonction de la concentration en TCDD (-log M) (nombre de fois), l'induction de CAT en présence de TCDD seule (courbe ■ et de TCDD avec 10⁻⁶ M de Resvératrol (courbe Δ ).

L'examen des résultats obtenus montre l'effet inhibiteur de Resvératrol vis-à-vis de l'induction de la transcription des enzymes de phase I par TCDD.

La figure 1B donne l'inhibition par le Resvératrol de l'induction en % de CAT en fonction de la concentration en TCDD (-log M). On constate que l'inhibition observée est dose-dépendante.

### . Inhibition de l'induction de la protéine Cyp1A1 par la dioxine.

On mesure l'expression de la protéine CYP1A1 *in vivo* en réponse à une concentration efficace de TCDD (10⁻ ¹⁰ M), en présence ou en l'absence de Resvératrol.

Les cellules T-47D sont cultivées dans le DMEM additionné de 10 % de sérum de veau foetal et traitées 48h par l'éthanol seul (témoin), 10⁻¹⁰ M dioxine (TCDD), 10⁻⁶ M Resvératrol (ResV), une combinaison de dioxine et Resvératrol (TCDD + ResV), 10⁻⁶ M α-naphthoflavone (α-NF) ou une combinaison de dioxine et α-naphthoflavone (TCDD + α-NF). Les cellules sont collectées et lavées dans le PBS, puis lysées par fusion-congélation répétée en conditions isotoniques. 75 µg de protéines sont analysées par électrophorèse en gel de polyacrylamide en conditions dénaturantes. Le gel est transféré sur une membrane de nylon. La membrane est saturée avec 5 % de lait écrémé et incubée avec un anticorps polyclonal de lapin (Daiichi Pure Chemicals Co, Japan) contre Cyp1A1. Les immuncomplexes sont détectés par chimioluminescence avec le kit Renaissance (DuPont).

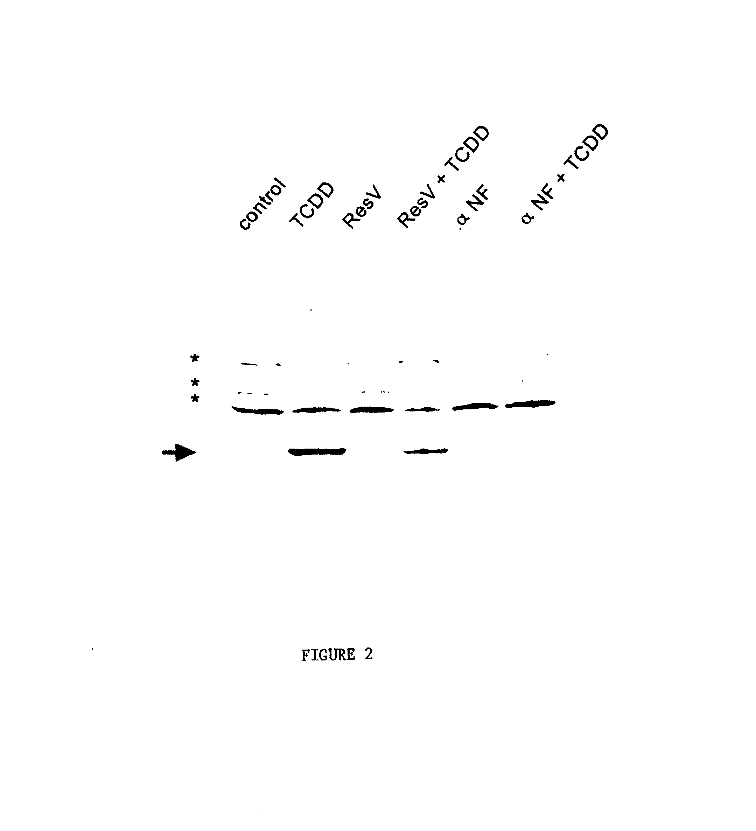

La figure 2 correspond à la photo du Western blot.
La flèche montre la bande spécifique de Cyp1A1, les astérisques signalent des bandes non-spécifiques.

On observe que le Resvératrol à une concentration de 10⁻⁶ M inhibe l'induction de la protéine CYP1A1 d'environ 60%.

L'effet inhibiteur du Resvératrol est légérement inférieur à celui de l'antagoniste classique de la dioxine, l'alpha-7,8-benzoflavone (ou α-naphthoflavone; αNF). En revanche, ce dernier composé, à la différence du Resvératrol, présente des effets agonistes autant qu'antagonistes à la concentration de 10⁻⁵M alors que le resvératrol est un pur antagoniste.

Ces résultats confirment que le Resvératrol est capable de bloquer l'induction de gènes sensibles aux ligands AhR, tels que CYP1A1/AHH et les autres enzymes de phase I.

### . Etude de l'activité antagoniste du Resvératrol

On a mesuré la capacité du Resvératrol de déplacer la dioxine radioactive (dioxine [2,6, di³H], 28 Ci/mmole, Terrachem, Kansas, USA) de son récepteur en comparaison avec d'autres ligands tels que l'a-NF ou l'indole-3-carbinol. On a utilisé un cytosol de foie de lapin pour ces études de compétition, car le lapin possède un récepteur AhR dont l'affinité est identique à celle du récepteur humain, à la différence du récepteur de souris.

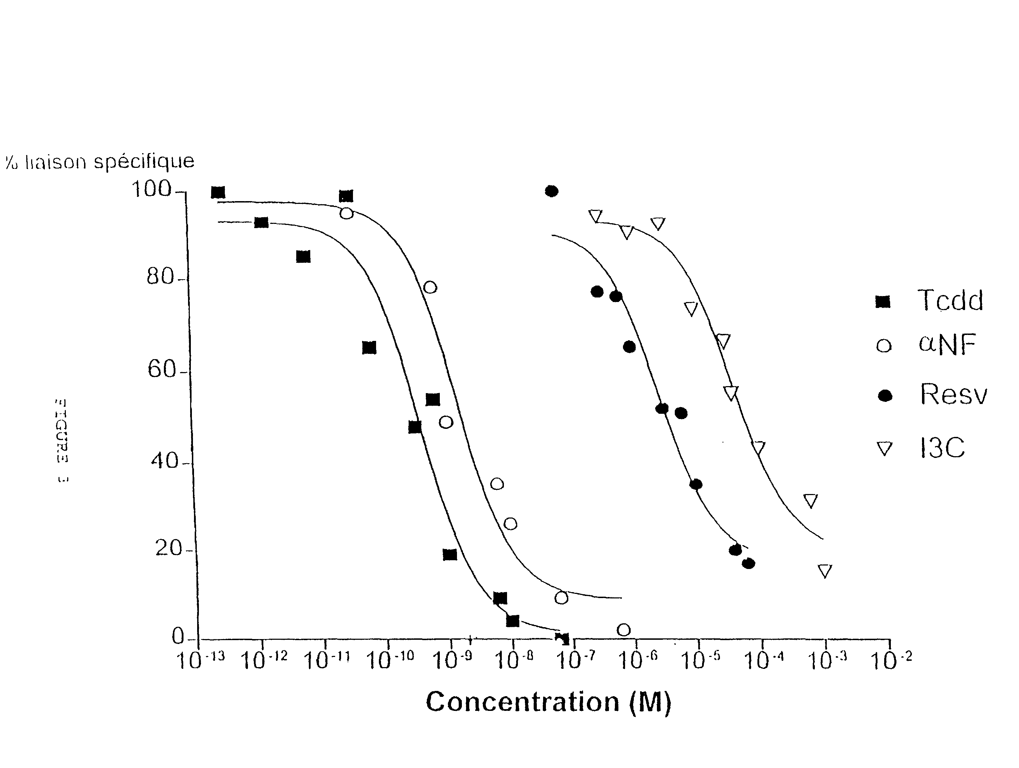

On rapporte sur la figure 3 le % de liaison spécifique au récepteur AhR en fonction de la concentration (M) en dioxine marquée au tritium ( ■ ), en présence de concentrations croissantes de α-naphtoflavone (o), de Resvératrol ( ● ), et d'indole-3-carbinol (¹³ C).

On observe que dans ce modèle, la dioxine et l'α-NF déplacent la dioxine marquée de son récepteur avec un Kd apparent de 3.10⁻⁹ M. Le Resvératrol déplace aussi la dioxine, bien qu'avec une affinité plus faible (6.10⁻⁶M). Le Resvératrol montre une affinité plus forte que l'autre ligand naturel, l'indole-3-carbinol. Ces résultats démontrent que le Resvératrol exerce ses effets antagonistes par compétition pour le récepteur AhR.

### . Etude d'effets de type stéroidien

On a transfecté de façon transitoire les cellules T-47D avec différentes constructions CAT sous contrôle de l'élément de réponse à l'estradiol (ERE) du gène de la vitellogénine ou celui, plus faible, du gène du récepteur de la progestérone, ainsi que des éléments de réponse à la progestérone (PRE) et aux androgènes (ARE) du virus murin MMTV et d'un élément de réponse à la vitamine D (VDRE) synthétique.

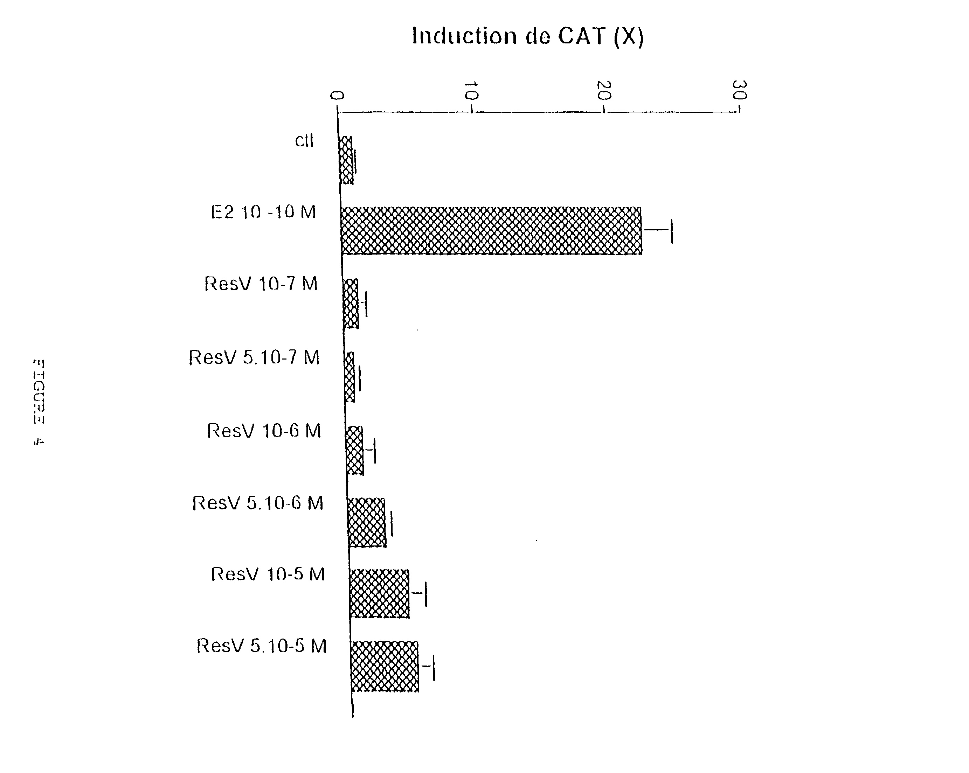

La figure 4 donne l'induction de CAT pour l'oestradiol (E2) 10⁻¹⁰ M, le Resvératrol (ResV) 10⁻⁷ M, 5 x 10⁻⁷ M, 10⁻⁶ M, 5 x 10⁻⁶ M, 10⁻⁵ M et 5 x 10⁻⁵ M.

Le Resvératrol n'a exercé aucun effet sur les PRE, ARE ou VDRE. Le Resvératrol a présenté un faible effet estrogénique sur l'ERE du gène de la vitellogénine, équivalent à 1/500.000 ème de l'effet de l'estradiol. La courbe dose-réponse montre qu'on observe à partir de 10⁻⁵ M de Resvératrol un effet équivalent au 1/5ème de celui de 10⁻¹⁰ M d'estradiol. Cette concentration d'estradiol est celle retrouvée dans le sang des humains de sexe masculin.

### . Régulation de l'activité NAD(P)H quinone réductase par la dioxine et ses antagonistes dans les cellules T-47D.

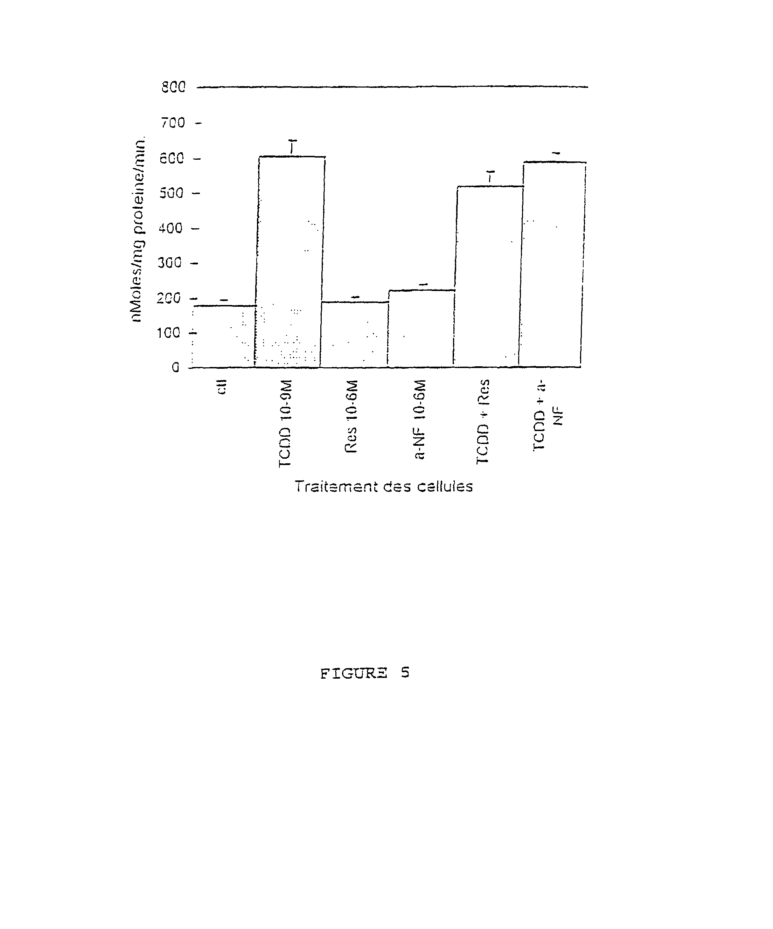

Les cellules T-47D ont été cultivées pendant 72 h en l'absence (ctl) ou en présence de dioxine 10⁻⁹ M (TCDD), Resvératrol 10⁻⁶ M (Res), α-naphthoflavone 10⁻⁶ M (α-NF), isolément ou selon les combinaisons indiquées. L'activité NQOR correspondante exprimée en nanomoles de cytochrome c réduit, par mg de protéines, par min, est donnée sur la figure 5.

Il est intéréssant de noter que ni le Resvératrol ni l'α-NF ne répriment l'induction indirecte des enzymes de phase II provoquée par la dioxine ou la NAD(P)H quinone réductase. Cet effet bénéfique est ainsi conservé.

Des résultats similaires sont obtenus pour la GST-Ya.

### . Etude de l'inhibition par le Resvératrol de l'induction d'autres gènes par les ligands AhR.

On a recherché, dans les banques de données EMBL et GENBANK, les gènes humains contenant des DRE dans leurs régions promotrices, en utilisant les consensus T/GNGCGTG dans les deux orientations possibles. En dehors des gènes des enzymes de phase I, on a découvert une population restreinte de gènes contenant un ou plusieurs DRE et donc susceptibles d'être régulés par les ligands AhR et le Resvératrol.

Ces gènes sont présentés dans les tableaux 1, 2 et 3 en fonction de leur association à des mécanismes physiologiques ou des états pathologiques distincts.

Le tableau 1 montre les gènes impliqués dans les régulations hormonales, le métabolisme des lipides, les maladies génétiques ou dégénératives, les cancers.

Le tableau 2 montre les gènes des cytokines et de leurs récepteurs qui sont impliqués dans le chimiotactisme et l'inflammation.

Le tableau 3 montre les virus et certaines protéines cellulaires qui interagissent avec eux.

**Tableau 1**

| GENES | Numéro d' ACCESSION | SITES (Nbre) | LOCALISATION | COMMENTAIRE |
|---|---|---|---|---|
| | | | | |
| Récepteur des Glucocorticoides | U10430 | 8 | promoteur | |

| **Enzymes** | | | | |
|---|---|---|---|---|
| NOS inductible | X97821 | 4 | promoteur | |
| NOS endothéliale | U24214 | 1 | promoteur | |
| Cyclooxygénase 2 | U20548 | 1 | promoteur | |
| PG H Synthase | U44805 | 1 | promoteur | |
| PG endoperoxide synthase | M31812 | 1 | promoteur | |
| 5-Lipoxygénase | M38191 | 5 | promoteur, cds | |
| 15-lipoxygénase | U63384 | 1 | promoteur | |
| Phospholipase A2 | U11239 | 2 | promoteur | |
| Thromboxane A2 récepteur | D15054 | 1 | promoteur | |

| **Récepteurs** | | | | |
|---|---|---|---|---|
| PDGF chaine A | U40769 | 2 | promoteur | |
| IGF-1 recepteur | M69229 | 6 | promoteur | |
| VEGF recepteur | D64016 | 2 | promoteur | |

| **Gènes impliqués en pathologie humaine** | | | | |
|---|---|---|---|---|
| Protéine APC | U02509 | 2 | promoteur, cds | polypes du colon |
| Protéine APC | D13981 | 1 | exon supplémentaire | spécifique du cerveau |
| Protéine APC | D13980 | 1 | 5'UTR | multiple variants |
| Prion Humain | D00015 | 2 | cds | |
| [Prion bovin] | D26150 | 1 | intron 1 | gène bovin |
| Ob(leptine) | U43589 | 2 | promoteur | obésité |
| préséniline | L76518 | 1 | promoteur | Alzheimer |
| GADD p153 | S40707 | 2 | promoteur | lésions de l'ADN |

| **Oncogènes** | | | | |
|---|---|---|---|---|
| WT 1b | S77896 | 1 | promoteur | Tumeurs de Wilm |
| WNT-5A | U39837 | 3 | promoteur | |
| PCNA | J05614 | 1 | promoteur | |
| p53 | J04238 | 17 | complete gene | apoptose et cycle cellulaire |
| p53 | J04238 | 1 | promoteur | |
| c-Ha-Ras | M13221 | 1 | promoteur | |
| c-jun | U60581 | 3 | promoteur | |
| c-myc p64 | M13930 | 4 | promoteur | |
| c-raf | M38134 | 2 | promoteur | |
| ATF 3 | U37542 | 2 | promoteur | |
| Tableau 1: Gènes humains contenant des DRE dans leur séquences régulatrices. Le gène du Prion bovin est aussi mentionné. Cds: séquence codante. UTR: région non traduite. | | | | |

**Tableau 2**

| GENES | Numéro d' ACCESSION | LOCALISATION | SITES (Nbre) | COMMENTAIRE |
|---|---|---|---|---|
| **Interleukines, Chemokines** | | | | |
| TNF | E01052 | cds | 1 | |
| IL 7 | M29048 | promoteur | 1 | |
| IL 10 | X73536 | promoteur | 1 | |
| IL1-RA | U65590 | gene (introns) | 4 | |
| RANTES | S64885 | promoteur | 1 | |
| LIGHT | AF03581 | cds | 1 | tnf-like |
| LIT | AF033854 | cds | 1 | |
| CD40 ligand | D31793 | promoteur | 1 | gp39 tnf-like |
| SCM 1 alpha | D63790 | cds | 2 | |
| MDC | U83171 | cds | 1 | |
| Eotaxine | U46572 | cds | 1 | chemokine |
| STCP-1 | U82992 | cds | 2 | T-cell chemokine |
| Netrine-2 like | U86758 | cds | 16 | |

| **Récepteurs** | | | | |
|---|---|---|---|---|
| IL-1 récepteur 1 | AF054830 | cds | 1 | |
| IL-1 récepteur 2 | X59770 | cds | 1 | |
| IL-2 récepteur a | U57613 | promoteur | 3 | |
| IL-2 récepteur b | X53093 | promoteur | 1 | |
| IL-8 récepteur a | U11870 | promoteur | 3 | |
| IL-8 récepteur b | U11866 | promoteur | 1 | |
| IL 10 récepteur | U00672 | cds | 1 | |
| CXCR4 | AF005058 | promoteur, cds | 9 | HIV co-récepteur |
| DARC | Y14873 | 5'UTR | 3 | |
| CCR6 | U45984 | cds | 1 | |
| TNF récepteur p75/80 | U53483, | promoteur, | 6 | |
| | S63368 | cds | | |
| RANTES/MIP1 récepteur | L10918 | cds | 2 | |
| DR3 | AF026070 | cds | 1 | tnf-like récepteur |
| decoy récepteur 2 | AF029761 | 5'UTR | 1 | tnf-like récepteur |
| interferon γ récepteur 2 | U68755 | promoteur | 3 | |
| VEGF récepteur | D64016 | promoteur | 2 | |

| **Protéines d'Adhésion** | | | | |
|---|---|---|---|---|
| connexine 32 | L47127 | 5'UTR | 1 | |
| connexine43 | U64573 | promoteur, cds | 3 | |
| ICAM (CD54) | M65001 | promoteur. cds | 4 | |

| **Gènes en relation.** | | | | |
|---|---|---|---|---|
| Ig E | M55420 | cds | 2 | région variable |
| IgE | S71428 | cds | 4 | isoforme |
| CD23 p45 | X06049 | promoteur | 1 | IgE récepteur |
| NF-IL3 | S79880 | cds | 1 | |
| NF Kappa B p65 | L01459 | promoteur | 2 | |
| NF Kappa B p50 | S57113 | promoteur | 1 | |
| I kappa B alpha | U08468 | promoteur | 2 | |
| RANKL | AF0190047 | cds | 1 | |
| Tableau 2: Gènes associés au chimiotactisme et à l'inflammation et contenant des DRE dans leur régions régulatrices. Cas: séquence codante. UTR: région non traduite. | | | | |

**Tableau 3**

| VIRUS HUMAINS et gènes associés | Numéro d' ACCESSION | LOCALISATION | SITES (Nbre) | COMMENTAIRE |
|---|---|---|---|---|
| | | | | |
| Cytomegalovirus IE-1 | M64941 | promoteur | 1 | |
| HTLV-1 | S76263 | 5'LTR | 1 | |
| HTLV-1 | S76263 | gène TAX | 1 | |
| HTLV-4 | X06392 | 5'LTR | 2 | |
| HIV 1 isolat BRU (LAV- | K02013 | 3' LTR | 1 | présent dans tous les |
| 1) | | | | isolats |
| HIV 2 | U22047 | génome | 2 | |
| HIV2 EHO | U27200 | génome | 1 | |
| CD4 | U01066 | promoteur | 1 | HIV récepteur |
| CD4 | S79267 | cds | 2 | HIV récepteur |
| CXCR4 | AF005058 | promoteur, cds | 9 | HIV co-récepteur |
| Papilloma Virus (HPV) | X52061 | génome lcr | 1 | "long control région" |
| HPV 11 | J04351 | génome | 2 | |
| HPV 16 | M33616 | génome | 1 | région 3' |
| HPV 25 | D50264 | promoteur | 1 | gène E6 |
| HPV 33, 47, 58 59 | | génome | | |
| Poliovirus 1 | X70509 | 5'UTR | 1 | |
| Rhinovirus 1B | D00239 | génome | 2 | |
| Rhinovirus 2, 14, 89 | | génome | | |
| Herpes virus HSV1 | U18080 | génome | 1 | IE transactivateur |
| Herpes virus HSV1 | M13885 | génome | 1 | segment "a" |
| Hepatite A virus | K02990 | génome | 1 | |
| Hepatite B virus | X98077 | génome | 1 | |
| Hepatite C virus | D30613 | génome | 4 | |
| Adenovirus type 2 | J01917 | génome | 21 | |
| Adenovirus type 12 | J01910 | génome | 1 | TR droit |
| Adenovirus type 12 | X14757 | promoteur | I | gène E1A |
| Adenovirus type 19A | X95259 | early region 3 | 1 | protéine liant les complexes HLA |
| Adenovirus type 41 | M18289 | génome | 1 | E1A gene |
| Virus syncitial respiratoire | M17212 | cds | 1 | protéine d'ancrage |
| Influenza virus, | | génome: sites | | Protéines M1, M2 |
| tous sous-types. | | multiple dans | | matrix, |
| | | divers gènes | | hémagglutinine, neuraminidase, ARN polymerase |
| Tableau 3: Génomes viraux ou gènes contenant des DRE's. UTR: région non traduite. LTR: longue région répétée terminale. IE: gènes d'expression immédiate précoce. Cds: séquence codante. TR: séquence répétée terminale | | | | |

Pour tester cette hypothèse, selon laquelle les gènes possédant des DRE dans leur promoteur seraient généralement sensibles aux ligands AhR et que cet effet pourrait être inhibé par le Resvératrol, on a choisi comme modèle représentatif le promoteur de la séquence répétée (LTR) de VIH-1, qui contient un DRE typique. On a transfecté les cellules T-47D avec une construction CAT contenant ce promoteur (-452/+77). Les cellules ont ensuite été traitées par la dioxine (10⁻⁹ M) ou le Resvératrol (10⁻⁶ M), seuls ou en association (p = 0,01).

La dioxine a induit le promoteur de VIH par un facteur de 3 et cette induction a été totalement supprimée par le Resvératrol.
Ces résultats démontrent donc que des concentrations de Resvératrol, de l'ordre de celles utilisables en thérapeutique humaine, sont capables de bloquer l'induction des enzymes de phase I et du promoteur du VIH par les ligands AhR. De la même manière, le Resvératrol s'avère capable de protéger contre d'autres effets transcriptionels pathologiques liés à l'exposition à ces substances nocives.

### . Etude du Resvératrol en tant qu'antagoniste des ligands AhR in vivo.

Des rats Sprague-Dawley femelle ont été traités par une combinaison de Benzol [a]pyrène et de DMBA (diméthylbenzanthracène), deux ligands AhR présents dans la fumée de cigarette. Des expériences préliminaires ont montré que cyp 1A1 était induit à partir de 1 mg/kg des deux ligands administrés ensemble. Ce traitement a ensuite été répété en présence ou absence de resvératrol à doses croissantes (1 et 5 mg/kg). Les animaux ont été sacrifiés et les organes prélevés. Des extraits cellulaires totaux ont été préparés et analysés par immunoblot pour détecter l'expression de cyp 1A1 dans ces tissus.

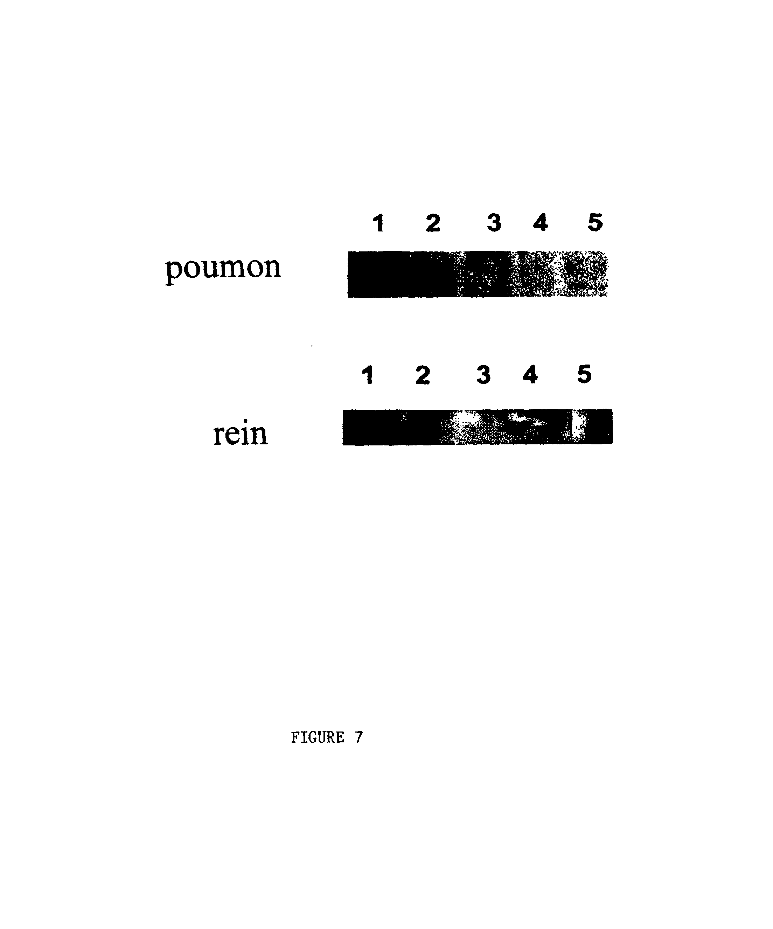

Les résultats obtenus sont rapportés sur la figure 7 où les lignes 1 à 7 correspondent respectivement à :
Ligne 1 : 1 mg/kg BaP/DMBA
Ligne 2 : 1 mg/kg BaP/DMBA plus 1 mg/kg resvératrol
Ligne 3 : 1 mg/kg BaP/DMBA plus 5 mg/kg resvératrol
Ligne 4 : 5 mg/kg resvératrol
Ligne 5 : véhicule seul (huile d'olive)
Chaque ligne contient 30 µg (poumon) ou 60 µg (rein) de protéines.

Comme le montre la figure 7, l'association BaP/DMBA provoque l'expression de cyp 1A1 dans le poumon et le rein. Cette induction est totalement supprimée par le resvératrol, dans les conditions de concentration égale des substances, montrant que le resvératrol est plus actif *in vivo* que *ex vivo*, où un excès de 5000 fois est nécessaire pour observer l'inhibition. Cette observation est en faveur d'une métabolisation du resvératrol en un composé plus actif dans l'animal, le resvératrol agissant ainsi comme une "pro-drogue".

### RÉFÉRENCES BIBLIOGRAPHIQUES

1/ Bock, K. W. (1994). Aryl hydrocarbon or dioxin receptor: biologic and toxic responses. Rev Physiol Biochem Pharmacol *125*, 1-42.
2/ Toborek, M., Barger, S. W., Mattson, M. P., Espandiari, P., Robertson, L. W., and Hennig, B. (1995). Exposure to polychlorinated biphenyls causes endothelial cell dysfunction. J Biochem Toxicol *10,* 219-226.
3/ Kleman M, Gustafsson J-A. (1996). Interactions of procarcinogenic heterocyclic amines and indolocarbazoles with the dioxin receptor. Biol Chem 377:741-762.
4/ Bartsch, H., Petruzzelli, S., De Flora, S., Hietanen, E., Camus, A. M., Castegnaro, M., Alexandrov, K., Rojas, M., Saracci, R., and Giuntini, C. (1992). Carcinogen metabolism in human lung tissues and the effect of tobacco smoking: results from a case-control multicenter study on lung cancer patients. Environ Health Perspect **98**, 119-24.
5/ Nebert, D. W., Puga, A., and Vasiliou, V. (1993). Role of the Ah receptor and the dioxin-inducible [Ah] gene battery in toxicity, cancer, and signal transduction. [Review] [75 refs]. Annals of the New York Academy of Sciences *685*, 624-40.
6/ Kerkvliet, N. I. (1995). Immunological effects of chlorinated dibenzo-p-dioxins. Environ Health Perspect *103 Suppl 9*, 47-53.
7/ Suskind, R. R. (1985). Chloracne, "the hallmark of dioxin intoxication". Scand J Work Environ Health *11*, 165-71.
8/ Mattison DR, Plowchalk DR, Meadows MJ, Miller MM, Malek A, London S. The effect of smoking on oogenesis, fertilization and implantation. Sem Reprod Health 7:291-304, 1989
9/ Rier SE, Martin DC, Bowman RE, Dmowski WP, Becker JL. (1993). Endometriosis in rhesus monkeys (Macaca mulatta) following chronic exposure to 2,3,7,8-tetrachlorodibenzo-p-dioxin. Fundamental & Applied Toxicology 21:433-41.
10/ Yao, Y., Hoffer, A., Chang, C. Y., and Puga, A. (1995). Dioxin activates HIV-1 gene expression by an oxidative stress pathway requiring a functional cytochrome P450 CYP1A1 enzyme. Environ Health Perspect *103*, 366-71.
11/ Burleson, G. R., Lebrec, H., Yang, Y. G., Ibanes, J. D., Pennington, K. N., and Birnbaum, L. S. (1996). Effect of 2,3,7,8-tetrachlorodibenzo-p-dioxin (TCDD) on influenza virus host resistance in mice. Fundam Appl Toxicol *29*, 40-7.
12/ Rowlands JC, Gustafsson J-A. (1997). Aryl hydrocarbon receptor-mediated signal transduction. Crit Rev Toxicol 27:109-34.
13/ Park, J. Y., Shigenaga, M. K., and Ames, B. N. (1996). Induction of cytochrome P4501A1 by 2,3,7,8-tetrachlorodibenzo-p-dioxin or indolo(3,2-b)carbazole is associated with oxidative DNA damage. Proceedings of the National Academy of Sciences of the United States of America *93*, 2322-7.
14/ Rimm EB, Klatsky A, Grobbee D, Stampfer MJ. (1996). Review of moderate alcohol consumption and reduced risk of coronary heart disease: is the effect due to beer, wine or spirits? Br Med J 312:731-6.
15/ Soleas GJ, Diamandis EP, Goldberg DM. (1997). Resveratrol: A molecule whose time has corne? And gone? Clin Biochemistry 30:91-113.
16/ Jang M, Cai L, Udeani GO, Slowing KV, Thomas CF, Beecher CWW, Fong HHS, Farnsworth NR, Kinghorn AD, Mehta RG, Moon RC, Pezzuto JM. (1997) Cancer chemopreventive activity of resveratrol, a natural product derived from grapes. Science 275:218-20.
17/ Stegeman JJ, Hahn ME, Weisbrod R, Woodin BR, Joy JS, Najibi S, Cohen RA. (1995) Induction of cytochrome P4501A1 by aryl hydrocarbon receptor agonists in porcine aorta endothelial cells in culture and cytochrome P4501A1 activity in intact cells. Molecular Pharmacology 47:296-306.
18/ Steinberg D, Parsatharathy S, Carew TE, Khoo JC, Witztum JL. (1989). Beyond cholestérol. Modifications of the low density lipoprotein that increase its atherogenicity. N Engl J Med 320:915-24.
19/ Rifkind AB, Gannon M, Gross SS. (1990). Arachidionic acid metabolism by dioxin-induced cytochrome P-450: a new hypothesis on the rôle of P-450 in dioxin toxicity. Biochem Biophys Res Commun 172:1180-88.
20/ Blaylock, B. L., Holladay, S. D., Comment, C. E., Heindel, J. J., and Luster, M. I. (1992). Exposure to tetrachlorodibenzo-p-dioxin (TCDD) alters fetal thymocyte maturation. Toxicol Appl Pharmacol *112*, 207-13.
21/ De Heer, C., Verlaan, A. P., Penninks, A. H., Vos, J. G., Schuurman, H. J., and Van Loveren, H. (1994). Time course of 2,3,7,8-tetrachlorodibenzo-p-dioxin (TCDD)-induced thymic atrophy in the Wistar rat. Toxicology & Applied Pharmacology *128*, 97-104.
22/ Yoo, B. S., Jung, K. H., Hana, S. B., and Kim, H. M. (1997). Apoptosis-mediated immunotoxicity of polychlorinated biphenyls (PCBs) in murine splenocytes. Toxicol Lett *91*, 83-9.
23/ Masten, S. A., and Shiverick, K. T. (1996). Characterization of the aryl hydrocarbon receptor complex in human B lymphocytes: evidence for a distinct nuclear DNA-binding form. Arch Biochem Biophys *336*, 297-308.
24/ Gehrs, B. C., Riddle, M. M., Williams, W. C., and Smialowicz, R. J. (1997). Alterations in the developing immune system of the F344 rat after perinatal exposure to 2,3,7,8-tetrachlorodibenzo-p-dioxin: II. Effects on the pup and the adult. Toxicology *122*, 229-40.
25/ Yang, Y. G., Lebrec, H., and Burleson, G. R. (1994). Effect of 2,3,7,8-tetrachlorodibenzo-p-dioxin (TCDD) on pulmonary influenza virus titer and natural killer (NK) activity in rats. Fundamental & Applied Toxicology *23*, 125-31.
26/ Kamath, A. B., Xu, H., Nagarkatti, P. S., and Nagarkatti, M. (1997). Evidence for the induction of apoptosis in thymocytes by 2,3,7,8- tetrachlorodibenzo-p-dioxin in vivo. Toxicol Appl Pharmacol *142*, 367-77.
27/ Yin, H., Li, Y., and Sutter, T. R. (1994). Dioxin-enhanced expression of interleukin-1 beta in human epidermal keratinocytes: potential rôle in the modulation of immune and inflammatory responses. Exp Clin Immunogenet *11,* 128-35.
28/ Fan, F., Yan, B., Wood, G., Viluksela, M., and Rozman, K. K. (1997). Cytokines (IL-1beta and TNFalpha) in relation to biochemical and immunological effects of 2,3,7,8-tetrachlorodibenzo-p-dioxin (TCDD) in rats. Toxicology *116*, 9-16.
29/ Gollapudi S, Kim CH, Patel A, Sindhu R, Gupta S. (1996). Dioxin activates human immunodeficiency virus-1 expression in chronically infected promonocytic U1 cells by enhancing NF-kappaB activity and production of tumor necrosis factor alpha. Biochem Biophys Res Commun 226:889-894.
30/ Takenaka, H., Zhang, K., Diaz-Sanchez, D., Tsien A. and Saxon, A. (1995) Enhanced IgE production results from exposure to the aromatic hydrocarbons from diesel exhaust: direct effect on B-cell production. J. Allergy Clin. Immunol. *95*, 103-115.
31/ Berkers, J. A., Hassing, I., Spenkelink, B., Brouwer, A., and Blaauboer, B. J. (1995). Interactive effects of 2,3,7,8-tetrachlorodibenzo-p-dioxin and retinoids on proliferation and differentiation in cultured human keratinocytes: quantification of cross-linked envelope formation. Arch Toxicol **69,** 368-78.
32/ Wanner, R., Panteleyev, A., Henz, B. M., and Rosenbach, T. (1996). Retinoic acid affects the expression rate of the differentiation- related genes aryl hydrocarbon receptor, ARNT and keratin 4 in proliferative keratinocytes only. Biochim Biophys Acta *1317*, 105-11.
33/ Franceschi S, Schinella D, Bidoli E, Dal Maso L, LaVecchia C, Parazzini F, Zecchin R. The influence of body size, smoking, and diet on bone density in pre-and postmenopausal women. Epidemiology 7(4):411-4, 1996
34/ Grisso JA, Kelsey JL,O'Brien LA, Miles CG, Sidney S, Maislin G, LaPann K, Moritz D, Peters B. Risk factors for hip fracture in men. Hip fracture study group. Am J of Epidemiology145(9):786-93, 1997
35/ Egger P, Duggleby S, Hobbs R, Fall C, Cooper C. Cigarette smoking and bone minerai density in the elderly. J or Epidemilogy & Community Health 50(1):47-50, 1996
36/ Hopper JL, Seeman E. (1994). The bone density of female twins discordant for tobacco use. N Engl J Med 330:387-392.
37/ Johansson C, Mellstrom D, An earlier fracture as a risk factor for new fracture and its association with smoking and menopausal age in women. Maturitas 24(1-2) :97-106, 1996.
38/ Safe, S., and Krishnan, V. (1995). Chlorinated hydrocarbons: estrogens and antiestrogens. Toxicol Lett *82-83*, 731-736.
39/ Abbott, B.D. (1995). Review of the interaction between TCDD and glucocorticoids in embryonic palate. Toxicology *105*, 365-73.
40/ Gurtoo, H. L., Williams, C. J., Gottlieb, K., Mulhern, A. I., Caballes, L., Vaught, J. B., Marinello, A. J., and Bansal, S. K. (1983). Population distribution of placental benzo(a)pyrene metabolism in smokers. Int J Cancer *31*, 29-37.
41/ Bilimoria, M. H., and Ecobichon, D. J. (1980). Responses of rodent hepatic, renal and pulmonary aryl hydrocarbon hydroxylase following exposure to cigarette smoke. Toxicology *15*, 83-9.
42/ Mattison DR, Thorgeirsson SS. Smoking and industrial pollution and their effects on menopause and ovarian cancer. Lancet 1:187-88, 1978
43/ Kiyohara, C., Hirohata, T., and Inutsuka, S. (1996). The relationship between aryl hydrocarbon hydroxylase and polymorphisms of the CYP1A1 gene. Japanese Journal of Cancer Research *87*, 18-24.
44/ Boyle P. (1997). Cancer, cigarette smoking and premature death in Europe: a review including the recommendations of European cancer experts consensus meeting, Helsinki, October 1996. Lung Cancer 17:1-60.

## Revendications

1. Utilisation de stilbènes polyhydroxylés, monomères ou polymères, ou des glycosides correspondants, ces composés étant sous forme racémique, ou d'isomères géométriques, pour la fabrication d'antagonistes de ligands du récepteur d'arylhydrocarbures pour traiter les pathologies induites par ces polluants.

2. Utilisation selon la revendications 1, **caractérisée en ce que** lesdits stilbènes sont trihydroxylés, et en particulier sont constitués par les 3, 5, 4'-trihydroxystilbène cis et trans ou resvératrols, et notamment par le trans resvératrol.

3. Utilisation selon la revendication 1, **caractérisée en ce que** lesdits stilbènes sont tétrahydroxylés et en particulier comprennent les 3, 4, 3', 5-tétrahydroxystilbènes ou picéatannols, et les 2, 3', 4, 5'-tétrahydroxystilbènes ou oxyresvératrols.

4. Utilisation selon la revendication 1, **caractérisée en ce que** lesdits stilbènes sont dihydroxylés et comprennent les 4, 4'-dihydroxystilbènes.

5. Utilisation selon l'une quelconque des revendications précédentes, **caractérisée en ce que** lesdits glycosides sont des glucosides, des galactosides, des mannosides.

6. Utilisation des antagonistes selon l'une quelconque des revendications 1 à 5, pour la prévention ou le traitement d'affections provoquées par l'exposition à des ligands arylhydrocarbures.

7. Utilisation des sitlbènes polyhydroxylés tels que définis dans l'une quelconque des revendications 1 à 5, pour la fabrication de médicaments à effet antagoniste de ligands du récepteur d'arylhydrocarbures, destinés à la prévention et au traitement de pathologies induites ou favorisées dans un contexte d'exposition à ces polluants arylhydrocarbures.

8. Utilisation selon la revendication 7, **caractérisée en ce que** lesdits médicaments se présentent sous une forme pour l'administration par voie orale, nasale, parentérale ou topique.

9. Utilisation selon la revendication 8, **caractérisée en ce que** lesdits médicaments se présentent sous forme de gélule, gouttes, sirop, sirop alcoolique, pour une administration par voie orale, de spray ou gouttes pour une administration par voie nasale, de solution pour une administration par voie parentérale et de crème, pommade, ou shampooing ou de lotion pour une application par voie topique, le véhicule comprenant une huile ou un alcool pharmaceutiquement acceptable.

10. Utilisation selon l'une quelconque des revendications 7 à 9, **caractérisée en ce que** lesdits médicaments sont administrables à raison de 0,1 mg à 5 g/jour, notamment de 20 à 200 mg/jour, et en particulier de 10 à 100 mg/jour.

11. Utilisation selon l'une quelconque des revendications 7 à 10, pour la fabrication de médicaments anti-viraux, pour prévenir les troubles associés aux infections virales liées à une exposition aux arylhydrocarbures, comme le rhume ou la grippe, ou pour traiter les polyposes nasales, pour traiter l'activation des infections virales latentes par les ligands AhR, telle que le SIDA induit par VIH.

12. Utilisation selon l'une quelconque des revendications 7 à 10, pour la prévention de l'encéphalite spongiforme causée par la protéine PrP (prion) chez l'humain et les bovins.

13. Utilisation selon l'une quelconque des revendications 7 à 10, pour la prévention de l'ostéoporose chez la femme à tous les âges de la vie et en particulier en post-ménopause et chez la femme âgée, dans le cas d'une exposition aux arylhydrocarbures.

14. Utilisation selon l'une quelconque des revendications 7 à 10, pour le traitement des états inflammatoires causés par la production excessive d'oxyde nitreux et/ou d'IgE, liée à une exposition aux arylhydrocarbures, notamment de dermatites atopiques, arthrite rhumatoïde, ostéo-arthrite, les maladies neurodégénératives (sclérose en plaques, sclérose amyotrophique) et diabète.

15. Utilisation selon l'une quelconque des revendications 7 à 10, pour réduire les états fébriles associés aux maladies infectieuses, virales et parasitaires, liées à une exposition aux arylhydrocarbures.

16. Utilisation selon l'une quelconque des revendications 7 à 10, pour le traitement des pathologies gynécologiques et endocriniennes telles que l'endométriose, les fibromes (léiomyomes), la pré-éclampsie et les avortements à répétition, liées à une exposition aux arylhydrocarbures.

17. Utilisation selon l'une quelconque des revendications 7 à 10, pour la prévention et le traitement de la maladie d'Alzheimer, liée à une exposition aux arylhydrocarbures.

18. Utilisation selon l'une quelconque des revendications 7 à 10, pour le traitement des dermatites, acné, lésions d'hyperkératose, eczéma, ainsi que le vieillissement de la peau et des rides, liées à une exposition aux arylhydrocarbures

19. Utilisation des antagonistes selon l'une quelconque des revendications 1 à 5 comme additifs alimentaires, en particulier pour supplémenter la nourriture des nouveaux nés, nourrissons et très jeunes enfants, dans un contexte d'exposition aux polluants arylhydrocarbures polycycliques et/ou halogénés.

20. Utilisation des antagonistes selon l'une quelconque des revendications 1 à 5, pour imprégner les filtres de cigarettes, afin de délivrer une dose d'antagoniste proportionnelle à la concentration de toxiques absorbée avec la fumée, et conduisant à une concentration sanguine permettant de bloquer l'induction de CYP1A1 et des autres enzymes de phase I au niveau des organes en contact avec les arylhydrocarbures.

## Claims

1. Use of monomers or polymers, polyhydroxylated stilbenes, or corresponding glycosides, these compounds being in racemic form, or in the form of geometric isomers, for making antagonists of arylhydrocarbon receptor ligands for treating pathologies induced by said pollutants.

2. Use according to claim 1, wherein said stilbenes are trihydroxylated, and particularly consist of cis and trans 3,5,4'-trihydroxystilbenes or resveratrol particularly trans resveratrol.

3. Use according to claim 1, wherein said stilbenes are tetrahydroxylated, and particularly comprise 3,4,3',5-tetrahydroxystilbenes or piceatannols, 2,3',4,5'-tetrahydroxystilbenes or oxyresveratrols.

4. Use according to claim 1, wherein said stilbenes are dihydroxylated and comprise 4,4'-dihydroxystilbenes.

5. Use according to anyone of the preceding claims, wherein said glycosides are glucosides, galactosides, mannosides.

6. Use of antagonists according to anyone of Claims 1 to 5, for the prevention or the treatment of pathologies induced by exposure to arylhydrocarbon ligands.

7. Use of polyhydroxylated stilbenes such as defined in anyone of claims 1 to 5, for making drugs with antagonist effects on arylhydrocarbon ligand receptors, intended for the prevention or the treatment of pathologies induced in a context of exposure to said arylhydrocarbon pollutants.

8. Use according to Claim 7, wherein said drugs are presented in a form for administration by the oral, nasal, parenteral or topical routes.

9. Use according to Claim 8, wherein said drugs are presented in the form selected from gel capsules, drops, syrup or alcohol syrup, for administration by the oral route, spray or drops for administration by the nasal route, solution for administration by the parenteral route, and cream, or ointment, shampoo or lotion for application by the topical route, the vehicle comprising an oil or a pharmaceutically acceptable alcohol.

10. Use according to anyone of Claims 7 to 9, wherein said drugs are administered at a dosage from 0.1 mg to 5 g/day, particularly from 20 to 200 mg/day, and in particular from 10 to 100 mg/day.

11. Use according to anyone of Claims 7 to 10, for making antiviral drugs, to prevent disorders associated with viral infections associated with exposure to arylhydrocarbons, such as the common cold or influenza, for treating nasal polyposis, for treating the activation of latent viral infections by AhR ligands, such as AIDS induced by HIV.

12. Use according to anyone of Claims 7 to 10, for the prevention of spongiform encephalitis caused by the PrP protein (prion) in man and in bovines.

13. Use according to any one of Claims 7 to 10, for the prevention of osteoporosis in women at all times of life, and particularly in post-menopause and elderly women, in a context of exposure to arylhydrocarbons.

14. Use according to anyone of Claims 7 to 10, for the treatment of inflammatory states caused by the excessive production of nitric oxide and/or of IgE, associated with exposure to arylhydrocarbons particularly atopic dermatitis, rheumatoid arthritis, osteoarthritis, neurodegenerative diseases (multiple sclerosis, amyotrophic sclerosis) and diabetes.

15. Use according to anyone of Claims 7 to 10, to reduce the febrile states associated with infectious, viral and parasitic diseases, associated to exposure to arylhydrocabons.

16. Use according to anyone of Claims 7 to 10, for the treatment of gynaecological and endocrine pathologies such as endometriosis, fibromas (leiomyomas), preeclampsia and habitual abortion, associated with exposure to arylhydrocarbons.

17. Use according to anyone of Claims 7 to 10, for the prevention and the treatment of Alzheimer's disease, associated with exposure to arylhydrocarbons.

18. Use according to anyone of Claims 7 to 10, for the treatment of dermatitis, acne, hyperkeratosis lesions, eczema, and also ageing of the skin and wrinkles, associated with exposure to arylhydrocarbons.

19. Use of the antagonists according to anyone of Claims 1 to 5 as food additives, particularly for supplementation of the food of new-borns, nursing infants and very young children, in the context of exposure to polycyclic and/or halogenated arylhydrocarbons pollutants.

20. Use of the antagonists according to anyone of Claims 1 to 5, to impregnate the filters of cigarettes, in order to deliver a dose of antagonist proportional to the concentration of the poisons absorbed with the smoke, and leading to a blood concentration making it possible to block the induction of CYP1A1 and other phase I enzymes at the level of the organs in contact with the arylhydrocarbons.

## Patentansprüche

1. Verwendung von monomeren oder polymeren polyhydroxylierten Stilbenen oder entsprechenden Glykosiden, wobei diese Verbindungen in racemischer Form vorliegen, oder geometrischen Isomeren zur Herstellung von Antagonisten von Liganden des Rezeptors für Arylkohlenwasserstoffe zur Behandlung von durch diese Schadstoffe induzierten Pathologien.

2. Verwendung nach Anspruch 1, **dadurch gekennzeichnet, daß** die Stilbene trihydroxylierte Stilbene sind und besonders die cis- und trans- 3, 5, 4' - Trihydroxystilbene, und insbesondere das Transresveratrol.

3. Verwendung nach Anspruch 1, **dadurch gekennzeichnet, daß** die Stilbene tetrahydroxylierte Stilbene sind und besonders die 3, 4, 3', 5 -Tetrahydroxystilbene oder Piceatanole und die 2,3',4,5' - Tetrahydroxystilbene oder Oxyresveratrole einschließen.

4. Verwendung nach Anspruch 1, **dadurch gekennzeichnet, daß** die Stilbene dihydroxyliert sind und die 4,4' - Dihydroxystilbene einschließen.

5. Verwendung nach einem der vorangehenden Ansprüche, **dadurch gekennzeichnet, daß** die Glykoside, Glucoside, Galaktoside, Mannoside sind.

6. Verwendung von Antagonisten nach einem der Ansprüche 1 bis 5 zur Vorbeugung vor oder Behandlung von Erkrankungen, die durch die Einwirkung von Arylkohlenwasserstoffliganden hervorgerufen werden,

7. Verwendung von polyhydroxylierten Stilbenen wie in einem der Ansprüche 1 bis 5 definiert zur Herstellung von Medikamenten mit antagonistischer Wirkung auf Liganden des Rezeptors von Arylkohlenwasserstoffen, wobei die Arzneimittel zur Vorbeugung vor oder Behandlung von Pathologien bestimmt sind, die im Rahmen einer Exposition gegenüber diesen Arylkohlenwasserstoff-Schadstoffen induziert oder begünstigt werden.

8. Verwendung nach Anspruch 7, **dadurch gekennzeichnet, daß** die Medikamente in einer für die orale, nasale, parenterale oder topische Verabreichung geeigneten Form vorliegen.

9. Verwendung nach Anspruch 8, **dadurch gekennzeichnet, daß** die Medikamente in Form von Gelatinekapseln, Tropfen, Sirup, alkoholischer Sirup für eine orale Verabreichung, in Form von Spray oder Tropfen für eine nasale Verabreichung, als Lösung für eine parenterale Verabreichung und als Creme, Pomade oder Shampoo oder Lösung für eine topischen Anwendung vorliegen, wobei das Vehikel ein Öl oder ein Alkohol umfaßt, die pharmazeutisch annehmbar sind.

10. Verwendung nach einem der Ansprüche 7 bis 9, **dadurch gekennzeichnet, daß** die Medikamente im Verhältnis 0,1 mg bis 5 g/Tag besonders 20 mg bis 200 mg/Tag und insbesondere 10 mg bis 100 mg/Tag verabreichbar sind.

11. Verwendung nach einem der Ansprüche 7 bis 10 zur Herstellung von Anti-Virus-Medikamenten zur Vorbeugung gegen Beschwerden, die mit Virusinfektionen verbunden sind, die mit einer Exposition gegenüber Arylkohlenwasserstoffen zusammenhängen, wie die Erkältung oder die Grippe, oder zur Behandlung von nasalen Polyposen, zur Behandlung der Aktivierung von latenten Virus-Infektionen durch die AhR-Liganden, wie das durch HIV induzierte AIDS.

12. Verwendung nach einem der Ansprüche 7 bis 10 zur Verhinderung von spongiformer Encephalitis, die durch das Protein PrP (Prion) bei Menschen und Rindern verursacht wird.

13. Verwendung nach einem der Ansprüche 1 bis 10 zur Verhinderung von Osteoporose bei der Frau in allen Lebensalterstufen und besonders in der Post-Menopause und bei der alten Frau im Fall einer Exposition gegenüber Arylkohlenwasserstoffen.

14. Verwendung nach einem der Ansprüche 7 bis 10 zur Behandlung von Entzündungszuständen, die durch übermäßige Produktion von Stickstoffoxid und/oder IgE verursacht sind, die zusammenhängt mit einer Exposition gegenüber Arylkohlenwasserstoffen, besonders atopische Dermatitis-Erkrankungen, rheumatische Arthritis, Osteoarthritis, die neurodegenerativen Erkrankungen (Plaque-Sklerose, amyotrophische Sklerose) und Diabetes.

15. Verwendung nach einem der Ansprüche 7 bis 10 zur Verringerung der Fieberzustände, die bei Infektions-, Virus- und Parasiten-Krankheiten auftreten, die mit einer Exposition gegenüber Arylkohlenwasserstoffen zusammenhängen.

16. Verwendung nach einem der Ansprüche 7 bis 10 zur Behandlung von gynäkologischen und endokrinen Pathologien, wie Endometriose, Fibrome (Leiomyome), Prä-Eklampsie und wiederholte Aborte, die mit einer Exposition gegenüber Arylkohlenwasserstoffen zusammenhängen.

17. Verwendung nach einem der Ansprüche 7 bis 10 zur Vorbeugung vor und Behandlung der Alzheimer-Krankheit, die mit einer Exposition gegenüber Arylkohlenwasserstoffen zusammenhägt.

18. Verwendung nach einem der Ansprüche 7 bis 10 zur Behandlung von Dermatitis-Erkrankungen, Akne, Hyperkeratose-Läsionen, Ekzema, sowie der Alterung der Haut und von Falten, die mit einer Exposition gegenüber Arylkohlenwasserstoffen zusammenhängen.

19. Verwendung von Antagonisten nach einem der Ansprüche 1 bis 5 als Lebensmittelzusatzstoffe besonders zur Ergänzung der Nahrung von Neugeborenen, Säuglingen und sehr jungen Kindern in einem Zusammenhang mit der Exposition gegenüber schädigenden polycyklischen und/oder halogenierten Arylkohlenw isserstoffen.

20. Verwendung von Antagonisten nach einem der Ansprüche 1 bis 5 zum Imprägnieren von Zigarettenfiltern, um eine Antagonisten-Dosis abzugeben, welche der Konzentration der mit dem Rauch absorbierten Giftstoffe proportional ist und zu einer Konzentration im Blut führt, welche die Blockierung der Induktion von CYP1A1 und anderen Enzymen der Phase I auf dem Niveau der mit den Arylkohlenwasserstoffen in Berührung kommenden Organe ermöglicht.
